# EUROPEAN PATENT APPLICATION

(11) **EP 1 947 455 A2**
(43) Date of publication of application: **23.07.2008**
(21) Application number: 07023062.8
(22) Date of filing: 28.11.2007
(51) Int. Cl.: G01N 33/03

(54) **A pattern recognition system for identifying a vegetable oil sample**

(30) Priority: 28.11.2006 EP 06024657
(71) Applicant: Montanuniversität Leoben, 8700 Leoben (AT)
(72) Inventor: Meisel, Thomas, 8700 Leoben (AT); Bandoniene, Donata, 8054 Graz (AT)
(74) Representative: Dilg, Haeusler, Schindelmann Patentanwaltsgesellschaft mbH

(57) **Abstract**

An apparatus for identifying a vegetable oil comprising sample, the apparatus comprising a database unit comprising a collection of data items each being characteristic for an assigned one of a plurality of vegetable oil comprising substances, an input unit adapted for receiving information regarding the vegetable oil comprising sample to be identified, and a determining unit adapted for identifying the vegetable oil comprising sample based on a pattern analysis of the received information in comparison to the collection of data items.

## Description

The invention relates to an apparatus for identifying a vegetable oil sample.

The invention further relates to a method of identifying a vegetable oil sample.

Moreover, the invention relates to a program element.

Further, the invention relates to a computer-readable medium.

D. Bandoniene, T. Meisel, S. Wagner, "ANALYSIS OF TRACE ELEMENTS IN PUMPKIN SEED OILS BY ICP-MS AS MEANS OF INDENTIFYING THE GEOGRAPHIC ORIGIN", 8th Symposium on instrumental Analysis, September 25 to 28, 2005 discloses that the authenticity and origin of food products are important from the standpoint of both commercial value and health aspects. The rapidly increasing progress of multi-element techniques in elemental analysis has lead to an increase of fingerprint analysis of food samples which allows to trace back the origin of the food sample (see N. Jakubowski, R. Brandt, D. Stuewer, H. R. Eschnauer and G. Görtges, Fresenius' Journal of Analytical Chemistry, 364, 1999, 424-428; S. Augagneur, B. Medina, J. Szpunar and R. Lobinski, Journal of Analytical Atomic Spectrometry, 11, 1996, 713-721).

Chondrite normalized rare earth elements (REE) patterns can be used in geochemistry as a fingerprint of the source. If the REE pattern in the soil is reflected in the vegetable oil and if the regional variations are significant enough, it could thus be possible to identify the provenance of the oil.

This may be used for the determination of the trace elements concentrations in pumpkin seeds and pumpkin seed oils by ICP-MS. Results are the basis to use their pattern as a fingerprint for the identification of pumpkin seed oils from different origins. A problem with the analysis of vegetable oil samples is the low trace elements content. ICP-MS excels by true multielement capabilities together with extremely low detection limits. D. Bandoniene et al. performed complete digestions with a high pressure asher (HPA, Anton-Paar, Graz) and analyzed the solutions with a quadrupole ICP-MS (HP4500 Agilent Technologies). For the determination of trace elements in particular REE several test samples of Styrian pumpkin seed oil from different Styrian oil mills were used.

Preliminary results for the elemental determination by the described procedure showed that each sample has significant differences in their trace element and REE concentration patterns. These differences likely reflect regional variations of the trace element distribution. Thus there is a probability for a reliable identification of the provenance of pumpkin seed oils.

However, it may be difficult to accurately determine an origin of a sample of vegetable oil based on a determined REE pattern.

It is an object of the invention to provide an accurate analysis system.

In order to achieve the object defined above, an apparatus for identifying a vegetable oil sample, a method of identifying a vegetable oil sample, a program element, and a computer readable medium according to the independent claims are provided.

According to an exemplary embodiment of the invention, an apparatus for identifying a vegetable oil comprising sample (such as a vegetable oil sample and/or a vegetable oil seed sample) is provided, the apparatus comprising a database unit comprising a collection of data items each being characteristic for an assigned one of a plurality of vegetable oil comprising substances (such as vegetable oils and/or vegetable oil seeds), an input unit adapted for receiving information regarding the vegetable oil comprising sample to be identified, and a determining unit adapted for identifying the vegetable oil comprising sample based on a pattern analysis of the received information in comparison to the collection of data items.

According to another exemplary embodiment of the invention, a method of identifying a vegetable oil comprising sample (such as a vegetable oil sample and/or a vegetable oil seed sample) is provided, the method comprising providing a database comprising a collection of data items each being characteristic for an assigned one of a plurality of vegetable oil comprising substances (such as vegetable oils and/or vegetable oil seeds), receiving information regarding the vegetable oil comprising sample to be identified, and identifying the vegetable oil sample based on a pattern analysis of the received information in comparison to the collection of data items.

According to still another exemplary embodiment of the invention, a program element (e.g. a software routine, in source code or in executable code) is provided, which, when being executed by a processor, is adapted to control or carry out a method of identifying a vegetable oil sample having the above mentioned features.

According to yet another exemplary embodiment of the invention, a computer-readable medium (e.g. a CD, a DVD, a USB stick, a floppy disk or a harddisk) is provided, in which a computer program is stored which, when being executed by a processor, is adapted to control or carry out a method of identifying a vegetable oil sample having the above mentioned features.

The sample investigation scheme according to embodiments of the invention can be realized by a computer program, that is by software, or by using one or more special electronic optimization circuits, that is in hardware, or in hybrid form, that is by means of software components and hardware components.

In the context of this application, the term "database" may particularly denote any computer file(s) or other collection of electronic data in which previously estimated data with regard to a type of vegetable oil (such as pumpkin seed oil) of different origins (such as different Styrian regions), countries (such as different European countries), plant type, agricultural years, etc. is stored in a computer-readable form. This database may be stored on a storage device like a harddisk or a CD and may be subsequently extended or updated.

The term "collection of data items" may particularly denote a set of structured data components which have a certain order to be accessible by a machine-based search or any other standardized algorithm. Individual data items may be grouped to data folders so as to manifest a certain correlation or assignment of the data. These data items may be related to vegetable oil of different kinds.

The term "information regarding the vegetable oil sample" may particularly denote data input to characterize a specific vegetable oil sample under test the origin of which shall be estimated. This information may include data indicative of trace elements being included in the sample, for instance a distribution of different ones of the rare earth elements (that is chemical elements having a partially filled 4f shell).

The term "pattern analysis" may particularly denote an analysis which is carried out by a computer or any other automatic processing unit and which compares or identifies patterns or motifs of the input information to corresponding patterns or motifs of data items of the database, for instance to look for matches between pattern in the vegetable oil sample and one pre-known vegetable oil included in the database. Such a pattern analysis may, for instance, include normalizing spectra, performing least square fits, identifying pre-known patterns of vegetable oils of different kinds or origins, etc. It may also include the analysis of a pattern of a sample under test with known motifs, for instance a positive or negative Cerium and/or Europium anomaly in a rare earth trace element pattern.

Particularly, the pattern analysis may include applying one or more pattern recognition algorithms. Pattern recognition (also known as classification, pattern classification or statistical classification) is a field within the area of machine learning and can be defined as "the act of taking in raw data and taking an action based on the category of the data". As such, it is a collection of methods for supervised learning. Pattern recognition aims to classify data (patterns) based on either a priori knowledge or on statistical information extracted from the patterns. The patterns to be classified may be groups of measurements or observations such as a trace element analysis of rare earth elements, defining points in an appropriate multidimensional space.

According to an exemplary embodiment of the invention, a database unit may include empiric or experimental information regarding a large number of different kinds of vegetable oils, for instance pumpkin seed oil from different origins. When the origin, quality, or any other characteristics of a vegetable oil sample under test shall be evaluated, chemical, physical and/or biological data relating to the sample (or even the sample itself) may be input to the system, and the system may performs a pattern recognition analysis by comparing the input information with the set of available data. One or more candidates in the collection of known vegetable oils matching properly to the vegetable oil sample are determined, and based on a statistical or numerical analysis, the best match or a number of best matches between the vegetable oil sample and one or more candidates for this oil sample may be identified. This may allow for a computer-based recognition of the origin of pumpkin seed oil samples, thereby for instance allowing to distinguish between original Styrian pumpkin seed oil and pumpkin seed oil from other geographic origins or from other quality levels.

Thus, the correctness of an indication of origin of a vegetable oil product may be verified. A correct classification may be confirmed, and an incorrect classification may be recognized. Further, the quality of the vegetable oil may be determined.

Embodiments of the invention may investigate vegetable oils (such as pumpkin seed oil) and/or vegetable oil seeds (such as pumpkin seeds). In other words, the analysis of seeds and/or seed oil may be used to identify the geographical origin of the seeds and/ or of the oil.

In order to receive the comparable/analyzable information regarding a vegetable oil sample to be identified, the sample may be brought in solution, for instance by an ashing procedure. This may be a more gentle treatment as compared to pressing pumpkin seeds in an oil mill, since the latter procedure may introduce impurities into the pumpkin seed oil which may deteriorate the analysis. Subsequently, this sample which is still, concerning its chemical composition, a proper fingerprint of the plant or plant seed from which the vegetable oil is usually derived, may then be analyzed by mass spectrometry or any other apparatus capable of quantitatively estimating the components of the sample.

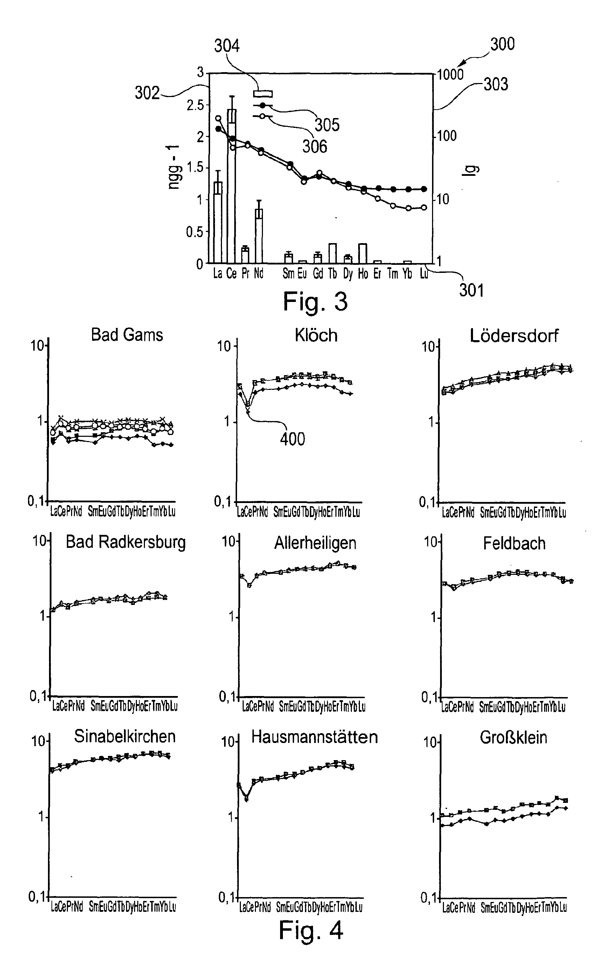

It has been found by the inventors that particularly a complete or partial trace element analysis of the rare earth elements are characteristic for the origin of a vegetable oil sample, and that a characteristic pattern in the distribution of the various rare earth elements (see Fig. 4) is a highly appropriate basis for performing an origin analysis by pattern recognition on the basis of a database search. Such rare earth elements do not contribute significantly to the biological function of a plant so that the pattern of rare earth elements may properly reflect properties of a soil on or in which the plant has grown. Furthermore, during an analysis of pumpkin seeds, which includes bringing the pumpkin seed oil sample in solution and analyzing it by means of a mass spectrometry device, a contamination during such a treatment is relatively unproblematic in the case of the rare earth elements.

Particularly, according to an exemplary embodiment of the invention, a database including information regarding pumpkin seed oil of known soils, seeds and/or oils may be used for analyzing a vegetable oil sample which is extracted in a gentle manner (for instance avoiding a pressing procedure). Such an unknown sample may then be analyzed chemically, physically and/or biologically. The database may be searched for a best match by carrying out a pattern recognition. In this context, exemplary pattern motivs are a gradient, an anomaly (for instance a Cerium anomaly), absolute concentrations, normalized concentrations, etc. of a trace element analysis. Particularly, a multi-variant (regression) analysis may be carried out. Elements of artificial intelligence, like Fuzzy logic and/or neural networks may be implemented. Program elements reflecting mathematical models may also be stored in a database for easier access. It is possible that the computer-based analysis may link the analysis to a statement regarding the quality of the vegetable oil under test. Therefore, an improvement of the quality of oils, a classification of oils, and the influence of trace elements on a sensory quality may be analyzed in more detail.

By implementing such an identification scheme in a computer system, huge amount of data may be evaluated which can no more be handled by a human being alone, for instance by a scientist. Hidden patterns or motifs being typical for specific vegetable oil types, for instance a Cerium anomaly, may be evaluated in a multi-dimensional data space, to find a best match in a specifically efficient manner by a computer system. This may allow to significantly improve reliability of measurement results and a strongly increased efficiency which would never be achievable without the database-based evaluation.

Regarding a method for estimating trace elements, it is possible to rely on a single method like mass spectrometry. However, in order to improve reliability and statistical accuracy of the result, a combination of different methods such as isotope dilution, mass spectrometry, a chromatographic separation using HPLC/MS, etc. may be carried out. Therefore, by combining several methods of analytically estimating the trace element characteristics of a vegetable oil sample, the analysis may be made more accurate and reliable. For ashing or mineralizing a vegetable oil sample, the original seeds, for example pumpkin seeds, may be treated with concentrated nitric acid at a temperature of 300 °C and a pressure of 120 bar. By such a treatment, it is believed that organic contributions may be converted at least partially into carbon dioxide, whereas inorganic contributions like rare elements may remain in the solution.

As an alternative to such an ashing unit, a microwave and ultraviolet light treatment may be used which might promote an automatization of the system.

Thus, according to an exemplary embodiment, a method for estimating the geographic origin of vegetable oils by fingerprinting with inorganic trace elements may be made possible. According to an exemplary embodiment, element trace distributions in vegetable oils may be examined for determining the geographical origin. The results regarding the distribution patterns, defined by exactly specified element ratios, may be collected in a database. For controlling the origin, the distribution patterns of element traces in commercial oils can be compared and identified with reference patterns in the database. Such a method may allow for accurately determining the origin of vegetable oils, for instance pumpkin seed oils, by pattern recognition of element traces, for example to protect a regional trademark and in order to prevent a wrong or misleading classification (for instance when non-Styrian pumpkin seeds are pressed and are classified as "original Styrian pumpkin seed"). Thus, embodiments of the invention may contribute to consumer protection.

In contrast to conventional systems, a fast, reliable and cost-efficient method for determining the origin of vegetable oils and/or oil seeds may be provided. The investigation of stable light isotopes in pumpkin seeds astonishingly results in a high reliability and reproducibility within climatically and geographically similar regions. The low cost involved for such a method may allow for a routine check of the origin of vegetable oils and oil seeds regarding their geographical origin.

For instance, concentrations or the distribution of rare earth elements in oil seeds and vegetable oils, particularly in pumpkin seeds and pumpkin seed oils, may provide a fingerprint characteristics for pumpkin seed oil of a specific origin. By analyzing a vegetable oil under examination on the basis of a rare earth element trace distribution in pumpkin seed oils for instance from Austria and other national/international regions in which pumpkins are cultivated, meaningful results may be obtained.

For pumpkin oil and pumpkin seeds, a system is provided which is capable of determining the origin using an isotope examination of pumpkin seed oils and/or pumpkin seeds. Generally, a distribution on the basis of isotopic patterns may be difficult due to tolerances even within one and the same region. However, geographically/geologically sensitive parameters like element trace composition, particularly a row of concentrations of different lanthanides, are of significant relevance in order to allow for a spatially resolved distribution. The trace element distribution is, in contrast to many organic components, strongly dependent of the composition of the soil, so that such an analysis may allow to distinguish even between spatially close regions. Such a trace element examination may substitute at least partially an isotope analysis and may allow to significantly reduce the costs. Thus, the fingerprinting origin method according to an exemplary embodiment of the invention may be cheaper, faster and more reliable when analyzing vegetable oils on the basis of inorganic trace elements.

Results of a chemical, biological and/or physical analysis may form the basis for the use of trace element patterns for identifying the different origin of different vegetable oil samples. This method may be particularly advantageously applied to pumpkin seeds and pumpkin seed oil. It may include a systematic examination of pumpkin seeds, pumpkin seed oils and soils at different regions. The individual samples may then be analyzed regarding their trace element concentrations. The measurement data may be evaluated mathematically (for instance may be normalized, and specific and characteristic element ratios may be calculated). The results may be introduced in a database in order to enable a regionally specific pattern recognition/fingerprint of samples under test.

Next, further exemplary embodiments of the apparatus will be explained. However, these embodiments also apply to the method, to the computer-readable medium and to the program element.

The vegetable oil may comprise at least one of the group consisting of pumpkin seed oil, olive oil, corn oil, sun flower oil, nut oil, and safflower oil. Such vegetable oils are specifically suitable for the database-based system according to exemplary embodiments of the invention, since a gentle treatment of the corresponding seeds may allow to maintain a pattern of 4f elements in such plants essentially unchanged. This may allow for a reproducible analysis even of unknown samples.

The analysis apparatus may comprise a user interface adapted for a bidirectional (data) communication with a user. The analysis apparatus may comprise a graphical user interface (GUI) via which a user may communicate with the system. This may include input elements like a keypad, a trackball, a joystick or even a microphone of a voice recognition system. Further, a display unit may be provided in the user interface like an LCD device, a TFT device, a plasma display or the like. Therefore, even a user without specific skills may operate the analysis system, for instance to verify a correctness of an origin of a product which has been bought by a consumer in a supermarket. However, it is also possible that a scientist operates the user interface to perform a sophisticated and detailed analysis of an unknown sample of vegetable oil.

The collection of data items may comprise data indicative of the vegetable oil (that is to say of the liquid oil sample extracted from a plant), data indicative of the plant from which the vegetable oil is extractable (that is to say geographical characteristics of a specific region, for example a Cerium anomaly which is known for pumpkin seed in different Styrian regions), data indicative of the soil on which the plant from which the vegetable oil is extracted is grown (for example a pattern of trace elements in the ground on which the pumpkin oil is grown) and/or data indicative of the country in which the plant from which the vegetable oil is extractable is grown (for instance characteristics for different countries like Austria, Poland and China). Thus, the collection of data items may be very large so that the computer skills of a high capacity of data throughput may be used efficiently to determine the origin of the vegetable oil sample.

The collection of data items may further comprise a trace element analysis, particularly an analysis of rare earth elements of the vegetable oil. Even if other trace elements may be used as well, it may be specifically preferred to analyze the lanthanides of a vegetable oil which are not involved significantly in biological processes and may therefore properly reflect a distribution of lanthanides in a soil. Therefore, patterns of soil, seeds and/or oil extracted from the seeds may be essentially similar which may be used for making the analysis more efficiently.

The determining unit may be adapted for identifying a most likely geographic origin, particularly a list of most likely geographic origins, of the vegetable oil sample based on a best match (or the best matches) between the received information and one of the data items during the pattern analysis. Therefore, the computer system may simply look for the best match between a pattern of a sample oil and one of the reference oils in the database. This may include performing a computer fit to determine which one of the pre-stored patterns is in best accordance with the pattern of the sample. However, in cases of doubts or when several patterns are in accordance with the pattern of the sample essentially equally well (that is to say when the deviation between the sample pattern and a plurality of reference patterns is lower than a user-defined or a priori defined threshold value), also a number of possible candidates may be extracted from the database. Such a purely numerical analysis may then be combined with the skills of a scientist operating the analysis apparatus, which scientist may for instance exclude one or more of the components of the list which may be excluded due to expert knowledge. Therefore, the automated system according to exemplary embodiments of the invention may be efficiently combined with human skills so as to efficiently use complementary information for a reliable determination of the origin of the vegetable oil sample.

The determining unit may be adapted for identifying the vegetable oil sample based on a gradient in a trace element analysis. For instance, a gradient and/or a curvature in a sequence of rare earth elements may be used as a meaningful parameter. Anomalies in a trace element analysis, for instance a typical dip in a Cerium region of the rare earth elements which may be found in many pumpkin seed oils may be used as well. A ratio between absolute values in a trace element analysis may be taken as a basis which may be more meaningful than the absolute value itself. Such a normalization may allow as well to derive hidden patterns. Furthermore, relative values in a trace element analysis may be used. For instance, a rare earth pattern may be normalized so that an area under the normalized pattern equals to a reference value. A multi-variant analysis may be carried out which may allow to derive meaningful results and to suppress artifacts. A predefined mathematical model which may be derived analytically, empirically or experimentally may be compared to the pattern of the sample under test. Elements of artificial intelligence such as Fuzzy logics and/or a neural network analysis may be included to combine the high mathematical capacity of a computer system with brain-like elements.

The analysis apparatus may comprise a classification unit adapted for classifying the identified vegetable oil, particularly for assigning the identified vegetable oil to belong to a class of vegetable oils (such as Styrian pumpkin seed oil) and/or for correlating a sensory quality with pattern in a trace element analysis. It is possible that the analysis result may not only be simply output for a user, but some kind of interpretation may be provided by the analysis system. This may include statements or predictions about qualities and correlations of recognized patterns with pre-known sensory perceptions of users. By taking such a measure, the computer may assist in the development of vegetable oils of high quality.

The analysis apparatus may comprise an analysis unit adapted for processing a supplied source (such as seeds or other plants) of the vegetable oil to thereby extract the vegetable oil to be identified. For example, pumpkin seeds may be supplied as a whole to the analysis unit which may then chemically treat the seeds so as to derive a sample of the vegetable oil from these pumpkin seeds having a trace element composition which substantially equals to those of the source, namely the seeds. Such a gentle treatment may suppress artifacts. Examples for the analysis unit are an ashing unit, a liquid chromatography device, a mass spectrometry device, a microwave source, and an ultraviolet light source. Particularly, a HPLC device and an MS device may be combined efficiently. In a similar manner, a microwave source and an ultraviolet light source may be efficiently combined to another analysis unit.

The analysis unit may be adapted for treating the supplied source of the vegetable oil by a combination of at least two independent treatment schemes to thereby redundantly determine the information regarding the vegetable oil sample to be identified. Such an analysis using a plurality of complementary methods may make the analysis more efficient and reliable, since errors typical for a specific method may be suppressed by the redundant use of a plurality of methods.

The aspects defined above and further aspects of the invention are apparent from the examples of embodiment to be described hereinafter and are explained with reference to these examples of embodiment.

The invention will be described in more detail hereinafter with reference to examples of embodiment but to which the invention is not limited.
Figure 1 illustrates a semi-automated analysis system according to an exemplary embodiment of the invention.
Figure 2 illustrates a fully-automated analysis system according to an exemplary embodiment of the invention.
Figure 3 illustrates a pattern of rare earth elements in a pumpkin seed oil mix.
Figure 4 illustrates patterns of rare earth elements in pumpkin seed oils of different origins.

The illustration in the drawing is schematically. In different drawings, similar or identical elements are provided with the same reference signs.

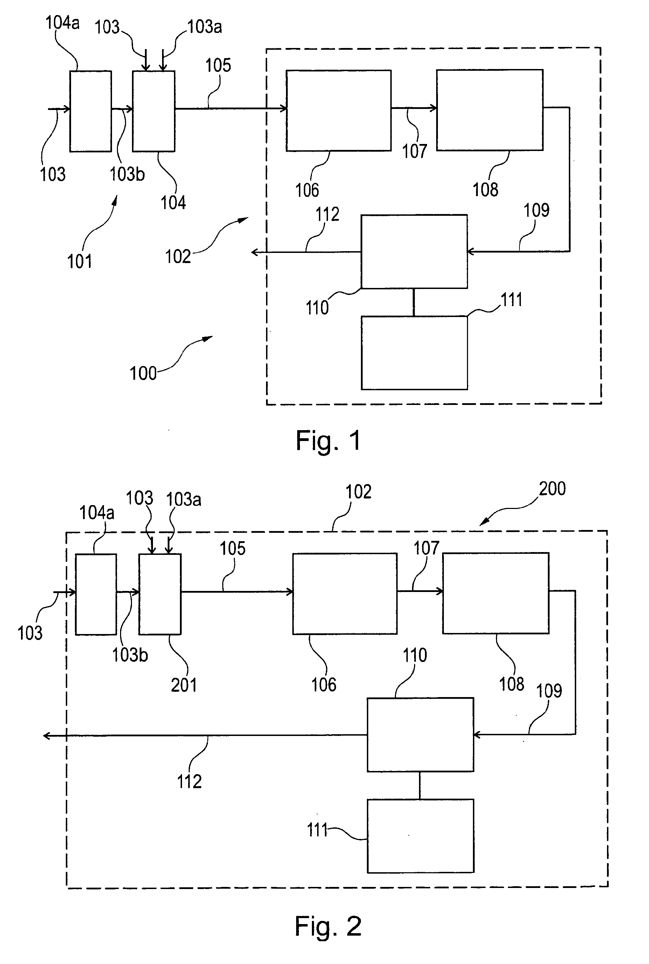

In the following, referring to **Fig. 1**, a computer-based system 100 for identifying a pumpkin seed sample or pumpkin oil samples according to an exemplary embodiment of the invention will be explained.

The system 100 comprises a component 101 which is to be operated by a user and furthermore includes an automated section 102 which can be operated and controlled by a computer system without the need of the assistance by a human being.

Pumpkin seeds 103 and/or pressed pumpkin seed oil 103a and/or extracted pumpkin seed oil 103b (which may be extracted by an extractor 104a) under test which are indicated schematically in Fig. 1 may be supplied to an ashing unit 104. In the ashing unit 104, the pumpkin seeds 103 and/or pressed pumpkin seed oil 103a and/or extracted pumpkin seed oil 103b may be treated chemically so as to obtain a pumpkin seed oil sample 105 (i.e. a fluidic solution of ashed pumpkin seeds or ashed pumpkin seed oil) to be analyzed. By the ashing procedure, the liquid oil sample 105 may be obtained on the basis of the pumpkin seeds 103 or oil 103a and 103b by a treatment in an acid milieu with a high pressure (for instance a high concentration of nitric acid at a pressure of 120 bar and a temperature of 300 °C). Such a treatment may ensure that the trace element distribution in the pumpkin seed oil 105 essentially equals to the trace element contribution in the pumpkin seed 103, since the introduction of inorganic impurities is avoided by the use of an ashing unit 104.

The fluidic sample 105 may then be supplied to a HPLC 106 (High Performance Liquid Chromatography apparatus). Within the HPLC 106, the fluidic sample 105 may be analyzed using one or more chromatographic columns to separate individual components including different trace elements of the pumpkin seed oil 105. The separated components of the treated sample 107 may then be supplied to a mass spectroscopy device 108 for further analysis. According to an exemplary embodiment, an ICP-MS, preferably an ICP-QMS (Q for quadrupole) may be combined with an IC or a HPLC.

At an output of the mass spectrometer 108, data 109 indicative of a trace element analysis of fluid pumpkin samples 105 of the pumpkin seeds 103 or pumpkin oils 103a and 103b is obtained and is supplied to a control unit 110. The control unit 110 may be a CPU of a computer system, a microprocessor, a separate computer, or the like. It may be capable of evaluating the data 110 indicative of fluid pumpkin samples 105 and to compare it to a collection of data items stored in an EEPROM 111 or any other memory. The EEPROM 111 may comprise a database which is accessible by the CPU 110 (in a read and/or write mode) and which comprises a collection of data items characteristics for pumpkin seed oil of different origins. On the basis of the information sources 109 and 111, the CPU 110 may determine a best match between characteristics 109 of the pumpkin seed oil 105 under test and one of the reference oils the characteristics of which being stored in the EEPROM 111.

The CPU 110 determines the best match(es) and outputs information 112 indicative of the most likely origin of the pumpkin seed oil sample 105 to a user, for instance to an input/output device or user interface (not shown).

**Fig. 2** shows an apparatus 200 according to another exemplary embodiment of the invention.

As can be taken from Fig. 2, all components of the apparatus 200 are included within the automated portion 102. In other words, the system 200 can be operated without the need of a human user to contribute her or his skills to the system. Simply providing the pumpkin seeds 103 (or already pumpkin seed oil 103a, 103b) to the apparatus 200 will result in an output 112 indicative of the identity or type of the source 103 (or 103a and 103b).

Also in Fig. 2, an automatic extractor 104a may be provided for extracting oil 103b from the seeds 103 before ashing/mineralizing. This allows for any opportunity: seeds directly, oil extracted from the seeds and oil directly from the bottle.

A main difference of the apparatus 200 and the apparatus 100 is that the ashing unit 104 is substituted by a microwave source and UV source unit 201. By treating the pumpkin seeds 103, pressed pumpkin oil 103a or extracted pumpkin oil 103b with a combined microwave and UV radiation in the unit 201, the oil sample 105 may be determined automatically without the need of a human user controlling an ashing unit 104. Therefore, Fig. 2 is an automated system which allows to supply the seeds 103 (or oils) at an input of the device 200 and to obtain information indicative of the origin 112 of the seeds 103 at an output of the device 200.

In the following, a more detailed operation of a system according to an exemplary embodiment of the invention will be given.

In a global trading system, in order to improve the quality of food stuffs, and due to different levels of legal regulations in different countries, the indication of the origin, and the reliability that food comes from a specific region is of increasing importance. In order to inform a consumer regarding a regional origin, the law of the different countries protect manufacturer's statements regarding the origin of food. This may allow a consumer to be aware of an origin of food which she or he consumes.

For example, a wrong classification of the origin of pumpkin seed oils can occur, or a mixture of cheap or low quality oils with oils of a specific origin may occur as well. Such events are recognizable, according to exemplary embodiments of the invention, for instance by legal authorities, the manufacturers of pure Styrian pumpkin seed oils and consumers. The Latin denotation *C*. *pepo var. styriaka* for Styrian pumpkins indicates the probable region of origin of this national mutation, namely the Steiermark. Since 1998, the European Union protects the Styrian pumpkin seed oil. In accordance with this protection, the use of the denotation "pure Styrian pumpkin seed oil" requires that this is 100% pure and results from a first pressing procedure in local companies. Embodiments of the invention may support in the determination whether a vegetable oil sample under test fulfils this regional requirement, or not.

According to an exemplary embodiment of the invention, a database system is provided in which results of a systematic investigation of soils, pumpkin seeds and pumpkin seeds oils of registered and non-registered regions in the Steiermark and in other Austrian federal countries, as well as in other countries from Eastern Europe, China and other countries are stored. Such a system may also ensure that quality standards of pure Styrian pumpkin seed oils are fulfilled. Exemplary embodiments of the invention provide an established method for a routine control of seeds within a company, for scientific purposes or in a store to enable a consumer to verify the origin of a product. Using such an analysis, it is easier and more reliable to determine an origin. This may include the recognition of an origin of the vegetable oil sample but also the recognition of falsifications of pumpkin seed oils and/or the mixture with cheap vegetable oils from other plants. Thus, improved quality control and the identification of falsifications may protect consumers. Therefore, exemplary embodiments of the invention provide an analysis method for controlling the purity of vegetable oils, in order to identify the addition of or the substitution by other oils in a qualitative and/or quantitative manner.

As a method for determining the origin of food, it is possible to analyze stable isotopic ratios. Such a method may include the measurement of the stable isotopic ratios of elements like hydrogen, carbon, nitrogen, oxygen and sulphur. It is also possible to consider the isotopes of strontium and lead. The analysis of isotopes of the main elements make the method very robust and forgery-proof, since an artificial modification is very difficult. The isotopic signature of a product may be the fingerprint of environmental and production-related conditions. Since each region is unique in a specific manner, the isotopic patterns of products of different origin are different. A determination of the geographic origin is therefore possible due to the different environmental parameters, like the composition of the regional rainfall, soil composition, etc.

Although a determination of pumpkin seed and pumpkin seed oil on the basis of the isotopic patterns is not always possible with high accuracy, it may be used according to exemplary embodiments and may be combined with additional geographically/geologically sensitive parameters in order to ensure an improved spatial resolution of the geographical distribution.

For this purpose, the identification of the origin of pumpkin seed/pumpkin seed oil may include an analysis of the rare earth elements in plant samples. Since a pattern of the rare earth trace elements is reflected in the soil and also in the plant serving as a source for the oil, and due to the presence of significant regional differences between the patterns, the inventors have recognized the possibility to use the rare element analysis for determining the origin of the oils. In addition to the regional differences, the content of the trace elements in pumpkin seed oils depends on many different factors. These factors (like homogeneity and geology of the soil, climate, influences of the seasons, contamination or loss of the analytes during processing may contribute to the unique fingerprint of pumpkin seed oils of different origins.

Falsifications of oils may be detected by analyzing the natural component composition of different oils. This may include a gas and/or high pressure liquid chromatographic determination of chemical components of different classes, for instance using a HPLC (High Performance Liquid Chromatography) analysis of the lipid acids and triglyceride compositions or the determinations of the phytosterole composition using GC-FID. However, this method may be complex and expensive.

According to an exemplary embodiment of the invention, such methods may be efficiently combined with or substituted by an analysis of the rare element composition of the samples, to increase the accuracy.

According to an exemplary embodiment of the invention, an analytic method for the determination of concentrations of trace elements may be provided, particularly of the rare earth elements in pumpkin seeds and pumpkin seed oils using ICP-MS (Inductively Coupled Plasma Mass Spectrometry). Investigations of the inventors have shown that pumpkin seed oils include a low concentration of particular rare earth elements. Analytical problems may be solved by the provision of a high sensitive and robust method which may include a HPLC- or IC-ICP-MS online coupling for a correct concentration determination, which may be implemented in routine analysis experiments.

A systematic examination of soils, pumpkin seeds and pumpkin seed oils of selected origins may allow to extend or update the database which may also allow to determine differences in pumpkin seed oil of different years. This may allow to ensure homogeneity of the soils and the quality over the years.

According to an exemplary embodiment, it is possible to identify the regional origin of the pumpkin seed oil and therefore to control to distinguish between domestic and foreign origin. This may be performed on the basis of a trace element pattern and/or by a combination with isotopic pattern (fingerprinting). Particularly, the trace element investigation may substitute the isotopic analysis partially or completely to increase accuracy and reduce costs.

Such analytical methods may be applied as well to other types of food such as other vegetable oils, particularly sunflower seeds and sunflower oils. Particularly, exemplary embodiments of the invention may correlate origin and quality and may examine quality criteria, for example:
- Soils (soil quality, homogeneity and geology)
- Climate (differences between trace element patterns from year to year)
- Influence of fertilizer
- Influence of processing in the oil mill (contaminations or losses of trace element)

Influences of such factors to the isotopic pattern and the trace element distribution in pumpkins, pumpkin seeds and pumpkin seed oils may be taken into account by the method. For instance, the a priori information that a reference vegetable oil has been processed in accordance with a specific method may be considered for the search for the best fit. For example, an averaging over a plurality of years, for instance 3 years, and/or an analysis regarding a single year may be performed or combined. Also expert knowledge regarding the distribution of isotopes and elements of the soil and the atmosphere into the plant may be considered for the evaluation.

It is also possible to investigate the influence of the trace element contents to the sensory quality (such as taste, smell and colour) of the oils. Pumpkin seed oils may comprise approximately 80% non-saturated fat acids, like oil or linol acid, which are very prone to oxidation. Specific metal ions like copper, iron, calcium, sodium, cobalt, nickel and magnesium may have different effects on the oxidative stability of the oils. Such metal ions may be easily introduced into the oil, thereby promoting a contamination during storage and processing. Such trace elements can accelerate the oxidation procedures in oils and may have a negative impact on the quality of the oils. The trace element composition and the sensory quality of the pumpkin seed oils may be considered during the evaluation according to exemplary embodiments of the invention. This may allow to determine proposals for an improved cultivation of oil pumpkins and a gentle processing to avoid contamination during the manufacturing procedure.

Embodiments of the invention may be based on the recognition that regional differences in the distribution of the rare earths and stable isotopes are reflected in the oils. The database may be continuously updated by a systematic investigation of pumpkin seeds, pumpkin seed oils and soils.

As a specifically appropriate analysis method, ICP-MS may be used for the exact determination of the concentrations of trace elements in pumpkin seeds and pumpkin seed oils. This may allow for a routine analysis, for instance a routine control of seeds in a company.

This may further allow to make the foreign or regional origin of the pumpkin seed oils or of the pumpkin seeds detectable by isotopic and/or trace element analysis. The developed method for the verification or determination of the origin of pumpkin seeds and pumpkin seed oil may be used to avoid that local origin denotations like "Styrian pumpkin seed oil" are wrongly used or are used in a misleading way.

By the fingerprinting of the trace element compositions, also mixtures between pumpkin seed oils with other plant oils, but also of pumpkin seed oils of different origin may occur.

Embodiments of the invention may also make predictions regarding the influence of the cultivation, cultivation regions and processing procedures to the quality of the pumpkin seed oil, thereby allowing to improve the quality of the pumpkin seed oil.

**Fig. 3** shows a diagram 300 illustrating a correlation between individual rare earth elements plotted along an abscissa 301 and a content plotted in ng/g along a first ordinate 302. Along a second ordinate 303, the content of the respective rare earth element per chondrite is plotted. A bar plot 304 is indicative of a seed oil mixture. A curve 305 is indicative of a sediment (chondrites normed), and a curve 306 is indicative of a seed oil mix x 50 (chondrites normed).

Fig. 3 clearly indicates that the rare earth distribution in a pumpkin seed oil (see curve 306) essentially equals to that in a sediment (see curve 305).

**Fig. 4** shows a plurality of diagrams indicative of the rare earth element patterns of pumpkin seed oil samples from different regions of the Steiermark.

The different measurements shown in each of the diagrams as compared to the different measurements in the different diagrams clearly show that there is a strong correlation between the origin and the pattern of the rare element distribution. Furthermore, differences between different regions are significant. Specific motifs in the diagrams, for instance the presence or absence of a Cerium anomaly 400 (that is to say a characteristic dip of some of the curves around Cerium) may be used as a fingerprint for a specific region.

Thus, diagrams in Fig. 3 and Fig. 4 show that the trace element distribution, particularly of the lanthanides or rare earth elements, are appropriate as a fingerprint of the origin of the pumpkin seed oils. As seen in Fig. 4, the oils of different Styrian oil mills are significantly different. For the estimation of the trace element concentration, particularly the rare earth elements, the pumpkin seed oils or pumpkin seeds, respectively, may be extracted using a high pressure ashing unit (HPA, Anton-Paar, Graz). Subsequently, the dissolved components may be analyzed using a quadrupole-ICP-MS device (HP4500 of Agilent Technologies). The trace element results obtained by these simple and fast methods show that the rare earth distribution in a pumpkin seed oil essentially equals to that of a sediment (Fig. 3).

The difference of trace element compositions in the different samples are large and very specific regarding the origin. Although the absolute concentration of these components are relatively low, in many cases less than 0.1 ng/g, sample specific differences are obvious. Without wishing to be bound to a specific theory, it is presently believed that these origins reflect the regional/geological variation of the plant regions (see Fig. 4).

By Soxhlet extraction of the grinded seeds with n-hexane or petroleum ether, a possible contamination of the oil with rare earth components during the pressuring procedure may be excluded. A faster and more cost-efficient alternative method to the soxhlet extraction is the fast and easily operable solvent extraction (using an ASE 100, Dionex). Due to the larger extraction efficiency of the ASE method, the rare element contents in the oils as determined may be higher.

It has further been determined that the isotopic patterns of ¹³C and ¹⁵N are dependent on the soil, fertilizer, climate (temperature, rainfall) and agricultural practice.

Therefore, a systematic examination under controlled conditions over a time of 3 years may allow to obtain a database including a plurality of parameters like element trace composition, analysis of other components (like pesticides), isotopic composition (light weight and radiogen) at identical soil, seed and oil samples. Particularly, a reference method may be provided for estimating the precise concentrations of rare earths using isotope dilution ICM-MS after a chromatographic separation and the application to plant samples.

Regarding ICP-MS, it is possible to analyze pumpkin seed oil using soxhlet extractions or accelerated solvent extraction (ASE 100, Dionex). Trace element analysis/rare element analysis of soils may include extracting a soil sample and treating it in an Na₂O₂ solution. Then, using ICP-Q-MS, the trace elements may be analyzed.

A high performance wavelength dispersing X-ray fluorescence spectrometer (RFA) may allow for an efficient and fast analysis with a minimal sample preparation (no extraction necessary), the calibration may be simple, and an analysis in a large concentration range may be possible.

It should be noted that the term "comprising" does not exclude other elements or features and the "a" or "an" does not exclude a plurality. Also elements described in association with different embodiments may be combined.

It should also be noted that reference signs in the claims shall not be construed as limiting the scope of the claims.

## Claims

1. An apparatus for identifying a vegetable oil comprising sample, the apparatus comprising
a database unit comprising a collection of data items each being characteristic for an assigned one of a plurality of vegetable oil comprising substances;
an input unit adapted for receiving information regarding the vegetable oil comprising sample to be identified;
a determining unit adapted for identifying the vegetable oil comprising sample based on a pattern analysis of the received information in relation to the collection of data items.

2. The analysis apparatus of claim 1,
wherein the vegetable oil comprising substances and/or the vegetable oil comprising sample comprise at least one of the group consisting of vegetable oil and vegetable oil seeds, particularly comprise at least one of the group consisting of pumpkin seed oil, olive oil, corn oil, sun flower oil, nut oil, and safflower oil.

3. The analysis apparatus of claim 1 or 2,
comprising a user interface adapted for a bidirectional communication with a user.

4. The analysis apparatus of any one of claims 1 to 3,
wherein the collection of data items comprises at least one of the group consisting of data characterizing a vegetable oil, data characterizing a plant from which a vegetable oil is extractable, data characterizing a soil on which a plant from which a vegetable oil is extractable is cultivated, data characterizing a country in which a plant from which a vegetable oil is extractable is cultivated, and data characterizing a region in which a plant from which a vegetable oil is extractable is cultivated.

5. The analysis apparatus of any one of claims 1 to 4,
wherein the collection of data items comprises a trace element analysis, particularly an analysis of rare earth elements, of the vegetable oil comprising substances.

6. The analysis apparatus of any one of claims 1 to 5,
wherein the determining unit is adapted for identifying a most likely geographic origin, particularly an ordered list of most likely geographic origins, of the vegetable oil comprising sample based on a best match, particularly an ordered list of best matches, between the received information and the data items during the pattern analysis.

7. The analysis apparatus of any one of claims 1 to 6,
wherein the determining unit is adapted for identifying the vegetable oil comprising sample based on at least one of the group consisting of a gradient in a trace element analysis, an anomaly in a trace element analysis, an absolute value in a trace element analysis, a ratio between absolute values in a trace element analysis, relative values in a trace element analysis, a multi-variant analysis, a mathematical model, a least squares fit, Fuzzy logics, and a neural network analysis.

8. The analysis apparatus of any one of claims 1 to 7,
comprising a classification unit adapted for classifying the identified vegetable oil comprising sample, particularly for assigning the identified vegetable oil comprising sample to a class of vegetable oil comprising substances and/or for correlating a sensory quality with a trace element analysis.

9. The analysis apparatus of any one of claims 1 to 8,
comprising a chemical analysis unit adapted for chemically treating a supplied source of the vegetable oil comprising sample to thereby determine the information regarding the vegetable oil comprising sample to be identified.

10. The analysis apparatus of claim 9,
wherein the chemical analysis unit comprises at least one of the group consisting of an ashing unit, a liquid chromatography device, a mass spectrometry device, a microwave source, and an ultraviolet light source.

11. The analysis apparatus of claim 9 or 10,
wherein the chemical analysis unit is adapted for treating the supplied source of the vegetable oil comprising sample by a combination of at least two independent treatments to thereby redundantly determine the information regarding the vegetable oil comprising sample to be identified.

12. A method of identifying a vegetable oil comprising sample, the method comprising
providing a database comprising a collection of data items each being characteristic for an assigned one of a plurality of vegetable oil comprising substances;
receiving information regarding the vegetable oil comprising sample to be identified;
identifying the vegetable oil comprising sample based on a pattern analysis of the received information in relation to the collection of data items.

13. A program element, which, when being executed by a processor, is adapted to control or carry out a method of claim 12 of identifying a vegetable oil comprising sample.

14. A computer-readable medium, in which a computer program is stored which, when being executed by a processor, is adapted to control or carry out a method of claim 12 of identifying a vegetable oil comprising sample.
